# EUROPÄISCHE PATENTSCHRIFT

(11) **EP 3 140 390 B1**
(45) Veröffentlichungstag und Bekanntmachung des Hinweises auf die Patenterteilung: **16.09.2020**
(21) Anmeldenummer: 15725487.1
(22) Anmeldetag: 07.05.2015
(51) Int. Cl.: C12M 3/06, C12M 1/12, C12M 1/42, C12M 1/34, C12N 5/071

(54) **HALBZEUG UND VORRICHTUNG ZUR IN-VITRO-HERSTELLUNG UND KULTIVIERUNG VON ZELLSCHICHTEN**
SEMI-FINISHED PRODUCT AND DEVICE FOR THE IN VITRO PRODUCTION AND CULTURING OF CELL LAYERS
DEMI-PRODUIT ET DISPOSITIF POUR LA PRODUCTION IN VITRO ET LA CULTURE DE COUCHES CELLULAIRES

(30) Priorität: 08.05.2014 DE 102014106423
(43) Veröffentlichungstag der Anmeldung: 15.03.2017
(73) Patentinhaber: Universitätsklinikum Jena, 07743 Jena (DE); Microfluidic ChipShop GmbH, 07747 Jena (DE)
(72) Erfinder: MOSIG, Alexander, 07743 Jena (DE); RENNERT, Knut, 07743 Jena (DE); KLEMM, Richard Robert, 07749 Jena (DE); GÄRTNER, Claudia, 07743 Jena (DE)
(74) Vertreter: Schaller, Renate
(86) Internationale Anmeldenummer: PCT/DE2015/100183
(87) Internationale Veröffentlichungsnummer: WO 2015/169287

(56) Entgegenhaltungen:
- WO-A-84/03047
- WO-A1-2012/032646
- WO-A1-2013/085909
- DE-A1- 3 923 279
- US-A1- 2007 212 773
- US-A1- 2011 136 218
- US-A1- 2014 038 279
- US-B1- 7 560 274

## Beschreibung

Die Erfindung betrifft ein Halbzeug und eine Vorrichtung zur In-vitro-Herstellung und Kultivierung von Zellschichten.

Zur In-vitro-Herstellung von Anordnungen von Zellschichten und deren Kultivierung sind verschiedene Verfahren und Vorrichtungen bekannt. Zellkultur-Inserts erlauben nur die Untersuchung von zellulären Prozessen unter statischen Bedingungen. Hierdurch kommt es zu einer Anreicherung von katabolischen Substanzen über den jeweiligen Zellkulturen, wodurch deren Wachstum und Vitalität eingeschränkt und zeitlich begrenzt wird. Da nur die Kultur unter statischen Bedingungen möglich ist, können keine Scherkräfte durch Flüssigkeiten an den Zellen erzeugt werden. Diese sind jedoch für eine Vielzahl von Zellen notwendig, damit diese physiologisch korrekte Eigenschaften, wie sie beispielsweise im menschlichen Körper auftreten, annehmen.

Bei der Boyden-Kammer können Zellen und Gewebe perfundiert werden, wodurch die Anreicherung katabolischer Substanzen vermieden, frische Nährsubstanzen den Zellen oder Geweben zugeführt und die beschriebenen Scherkräfte an den Zellen oder Geweben erzeugt werden können. Dieses System besitzt jedoch den entscheidenden Nachteil, dass die Zellen nur von einer Seite (meist apikal) effektiv vom Nährmedium perfundiert werden. Die Zellen können nicht basolateral perfundiert werden. Hierdurch werden das Zellwachstum und die Zellvitalität ebenfalls eingeschränkt und zeitlich begrenzt. Weiterhin ist das System auf die Kultur von maximal einer zweidimensionalen Zell- oder Gewebefläche begrenzt.

Die in der Schrift US 2011/0091930 A1 beschriebene Vorrichtung erlaubt aufgrund ihrer Konstruktionsmerkmale nur die Kultivierung von maximal einer Zellschicht. Zudem kann aufgrund der Konstruktion keine direkte Mikroskopie der Zellen unter Perfusionsbedingungen in der Kavität erfolgen, da versorgende Kanalsysteme unterhalb der Kulturfläche die optische Beobachtungsachse des Mikroskops behindern.

Die in der Schrift DE 10 2009 035 502 A1 beschriebene Vorrichtung erlaubt zwar die Beobachtung von Zellen und Geweben unter Flussbedingungen, allerdings lassen sich die Kavitäten nicht individuell durch getrennte Flüssigkeitsströme perfundieren. Zudem ist das System aufgrund seiner konstruktiven Merkmale nicht geeignet, mehrere Kulturflächen für Zellen in einer Kavität zur Verfügung zu stellen, wodurch die für eine lebensnahe Funktionsabbildung von Organen in vitro wichtige Interaktion zwischen verschiedenen Zellschichten nicht ausreichend berücksichtigt werden kann.

Die technische Lehre aus der Schrift US 8 357 528 B2 erlaubt die Kultivierung von sich gegenüberliegenden Zellschichten an einem porösen Gebilde wie beispielweise einer Membran. Die Membran trennt zwei Kulturkammern vertikal voneinander ab. Aufgrund des Aufbaus des Kammersystems ist beispielsweise eine direkte mikroskopische Beobachtung von adhärierenden und / oder transmigrierenden Zellen nicht möglich. Das System ist auf die Aufnahme von zwei Zellschichten beschränkt. Zur Abbildung einer mehrschichtigen Organoid-Struktur sind meist wenigstens drei Zellschichten notwendig.

Ebenfalls auf zwei Zellschichten sind Möglichkeiten begrenzt, die in den Schriften US 2007/0037277 A1, US 2009/0023608 A1 und US 2010/0216244 A1 beschrieben werden. WO013/085909 und WO2012/032646 offenbaren weitere Zellkulturvorrichtungen, die mindestens zwei durch eine Membran getrennte Kavitäten aufweisen, wobei jede Kavität eine Flüssigkeit-Einlassöffnung und eine Flüssigkeit-Auslassöffnung besitzt.

Der Erfindung liegt die Aufgabe zugrunde, eine weitere Möglichkeit zur In-vitro-Herstellung und Kultivierung von Zellschichten vorzuschlagen, durch welche Zellschichten in vorbestimmter Weise angeordnet, kultiviert und untersucht werden können. Es ist ebenfalls Aufgabe der Erfindung, eine Vorrichtung vorzuschlagen, mit der auch komplexe Anordnungen von Zellschichten kultiviert und untersucht werden können.

Die Aufgabe ist durch die Gegenstände der unabhängigen Ansprüche gelöst. Vorteilhafte Ausgestaltungen finden sich in den abhängigen Ansprüchen.

Mit einer erfindungsgemäßen Vorrichtung kann ein Verfahren ausgeführt werden, das die folgenden Schritte aufweist:
A Bereitstellen einer gegen ihre Umgebung bis auf eine Einlassöffnung und eine Auslassöffnung abgeschlossenen ersten Kavität mit einer ersten Wand als ein erstes Zellsubstrat und einer gegenüberliegenden und durch einen Spalt von der ersten Wand getrennten zweiten Wand als ein zweites Zellsubstrat,
B Ausrichten der ersten Kavität so, dass eine freie Oberfläche eines mit Zellen zu besiedelnden Zellsubstrats orthogonal zur Erdanziehungskraft orientiert ist,
C Einfüllen einer die Zellen enthaltenden Zellsuspension in die erste Kavität, Sedimentieren der Zellen auf dem zu besiedelnden Zellsubstrat und Adhärieren der Zellen auf dem zu besiedelnden Zellsubstrat, so dass eine erste Zellschicht gebildet wird,
D erneutes Ausrichten der ersten Kavität so, dass ein mit Zellen zu besiedelndes Zellsubstrat orthogonal zur Erdanziehungskraft orientiert ist, wobei das zu besiedelnde Zellsubstrat entweder das bereits besiedelte Zellsubstrat oder das andere Zellsubstrat ist,
E erneutes Einfüllen einer die Zellen enthaltenden Zellsuspension in die erste Kavität, Sedimentieren der Zellen auf dem zu besiedelnden Zellsubstrat und Adhärieren der Zellen auf dem zu besiedelnden Zellsubstrat, so dass eine weitere Zellschicht gebildet wird und
F Wiederholen der Schritte D und E, bis eine gewünschte Anordnung von Zellschichten in der ersten Kavität hergestellt ist.

Unter einer Kavität wird im Folgenden eine Vertiefung verstanden. Diese kann durch angrenzende Wände umschlossen sein und einen Raum bilden. Oberseiten der Wände bilden Ränder der Kavität.

Ein Zellsubstrat ist eine Oberfläche aus einem künstlichen oder natürlichen Material, an der Zellen adhärieren und bei entsprechender physiologischer Versorgung überleben können. Zellsubstrate sind beispielsweise Oberflächen aus Kunststoff, Metall oder Glas (künstliche Materialien). Zellsubstrate können aber auch Zellschichten, Gewebeschichten oder mit Biomolekülen, beispielsweise mit Biopolymeren, bedeckte Oberflächen (natürliche Materialien) sein. Zellsubstrate aus künstlichen Materialien können eine Oberflächenstrukturierung, beispielsweise eine Rauigkeit oder eine Beschichtung (z. B. anionisch/kationisch, hydrophob/hydrophil, Proteine, Polymere, Zellen), aufweisen, durch die ein Adhärieren begünstigt wird.

Sind die einander gegenüberliegenden Oberflächen der Wände voneinander getrennt und kann zwischen diesen ein gasförmiges oder flüssiges Medium, nachfolgend auch als Fluid bezeichnet, hindurchfließen, ist zwischen den beiden Wänden ein Spalt vorhanden. Dieser Spalt kann während der Durchführung des beschriebenen Verfahrens durch aufwachsende Zellschichten verringert werden.

Bei dem beschriebenen Verfahren werden die Zellen auf ein zu besiedelndes Zellsubstrat aufgebracht, indem die Wirkung der Gravitation genutzt wird. Die Zellen sedimentieren auf dem zu besiedelnden Zellsubstrat, wo sie adhärieren. Die adhärierten Zellen sind also an das Zellsubstrat gebunden und werden bei einem Abfließen der Zellsuspension und bei einem Wechsel in der Orientierung der Kavität an dem Zellsubstrat verbleiben.

Eine Zellschicht liegt vor, wenn wenigstens einige Zellen auf dem Zellsubstrat adhäriert sind. Das Zellsubstrat kann daher vollständig durch Zellen aus einer Zellsuspension bedeckt sein. Je nach den vorliegenden physiologischen Bedingungen, der Beschaffenheit des Zellsubstrats und der biologischen, biochemischen und biophysikalischen Eigenschaften der Zellen der Zellsuspension kann das Zellsubstrat auch nur zu einem Anteil, beispielsweise zu 5 %, 25 %, 50 % oder 75 %, der Oberfläche des Zellsubstrats bedeckt sein. Durch eine oder mehrere Zellschichten ist eine Zellkultur gebildet.

In einer Zellsuspension können Zellen eines Zelltyps vorliegen. Es ist in weiteren Ausgestaltungen des beschriebenen Verfahrens auch möglich, dass in einer Zellsuspension Zellen verschiedener Zelltypen vorliegen.

Durch das Verfahren wird es ermöglicht, Zellsubstrate mit Zellen zu besiedeln. Es können dabei sukzessive auf einem oder mehreren der Zellsubstrate Folgen von Zellschichten aufgebracht werden. Dadurch können die Kavitäten in vorbestimmter Weise mit Zellschichten ausgewählter Zelltypen in vorbestimmten Reihenfolgen der Schichten sowie in vorbestimmter Konfiguration der Zellschichten bzw. der Folgen von Zellschichten der verschiedenen Zellsubstrate gestaltet werden. Grenzen dieser Möglichkeiten liegen lediglich in dem verfügbaren Platz und in der Kompatibilität der Zellschichten untereinander, insbesondere dann, wenn eine Zellschicht als ein zu besiedelndes Zellsubstrat verwendet wird.

Günstig ist dabei, dass die Zellschichten innerhalb der Kavität auf die Zellsubstrate aufgebracht werden können. Es ist daher nicht erforderlich, die einzelnen Zellsubstrate separat zu besiedeln und dann erst die Kavität durch eine Montage herzustellen, indem beispielsweise die Zellsubstrate einander gegenüber positioniert werden. Durch das beschriebene Verfahren wird außerdem die Gefahr der Beschädigung oder der sonstigen physischen Beeinflussung der Zellschichten während der Montage vermieden.

Wie vorstehend bereits angesprochen, ist es möglich, dass in den Schritten C und E Zellsuspensionen mit Zellen verschiedener Zelltypen verwendet werden, sodass mindestens die erste und eine weitere Zellschicht durch Zellen verschiedener Zelltypen gebildet werden.

Das Verfahren kann vorteilhaft zur Herstellung von Organoiden verwendet werden. Organoide sind künstliche Nachbildungen von Organen oder von einzelnen Bereichen eines Organs, beispielsweise eines Lebersinusoids.

Dabei wird eine Vorrichtung, aufweisend eine erste Kavität und eine zweite Kavität, verwendet, wobei die zweite Kavität wie die erste Kavität zwei sich gegenüberliegende und durch einen Spalt voneinander getrennte Wände als je ein Zellsubstrat aufweist und die zweite Wand der ersten Kavität durch eine erste Seitenfläche einer porösen Membran gebildet ist und eine zweite Seitenfläche der Membran als eine Wand der zweiten Kavität dient. Es wird mindestens jeweils eine Zellschicht eines Zelltyps auf wenigstens zwei der Zellsubstrate hergestellt.

Die Erhaltung der biologischen Funktionalitäten der Zellschichten einer Anordnung von Zellschichten oder eines Organoids, die nach einem der vorstehend beschriebenen beschriebene Verfahren hergestellt sind, ist dadurch möglich, dass durch die erste Kammer ein erster Fluidstrom und durch die zweite Kammer ein zweiter Fluidstrom geleitet wird, wobei der erste und der zweite Fluidstrom Nährstoffe enthalten.

Nährstoffe sind beispielsweise Energieäquivalente, Zucker, ATP, NADP, NADPH, aber auch Wachstumsfaktoren, Hormone, Enzyme, synthetische Wirkstoffe und dergleichen.

Besonders vorteilhaft ist dabei, wenn eine Vorrichtung verwendet wird, deren zweite Wand sowie das Zellsubstrat dieser zweiten Wand durch eine poröse Membran gebildet ist. In diesem Fall kann eine perfusive Versorgung der Zellschichten auf der zweiten Wand durch die erste Kavität und durch die zweite Kavität erfolgen.

Die Strömungsverhältnisse in der ersten und in der zweiten Kavität sind durch die Auswahl der Form der Kavitäten beeinflussbar. So ist durch eine langgestreckte Form der ersten und der zweiten Kavitäten je eine laminare Strömung des ersten Fluidstroms durch die erste Kavität und des zweiten Fluidstroms durch die zweite Kavität erzielt.

Durch eine runde Form der ersten und der zweiten Kavitäten wird je eine turbulente Strömung des ersten Fluidstroms durch die erste Kavität und des zweiten Fluidstroms durch die zweite Kavität erzielt.

Die Strömungsverhältnisse sind zudem durch die Dimensionierung, die Anordnung und die Ausrichtung von Ein- und Auslässen an den Kavitäten beeinflussbar.

Es ist vorteilhaft ermöglicht, dass die stofflichen Zusammensetzungen des ersten und / oder des zweiten Fluidstroms gesteuert eingestellt werden und Messwerte an den Zellschichten erhoben werden.

Die Messwerte von chemischen und / oder physikalischen Parametern können mindestens an einem der ersten und zweiten Fluidströme erfasst werden. Dies erfolgt vorzugsweise bei deren Austritt aus der ersten bzw. aus der zweiten Kavität. Die Messwerte können dazu genutzt werden, die stofflichen Zusammensetzungen des ersten und / oder des zweiten Fluidstroms zu steuern, aber auch um Rückschlüsse auf die Vorgänge in den Zellschichten in den Kavitäten zu ziehen. Durch diese Möglichkeiten der Erfassung und Auswertung von Messwerten ist ein Testverfahren ermöglicht.

In einer weiteren Ausgestaltung des Verfahrens können die Messwerte mittels Impedanzmessungen erfasst werden.

Es ist ferner möglich, dass die Membran durch Einstellung unterschiedlicher Druckverhältnisse in der ersten und in der zweiten Kavität durchgebogen wird, wobei der Betrag der Durchbiegung und die Richtung der Durchbiegung durch die Einstellung der Druckverhältnisse gesteuert werden. Durch diese Möglichkeit können Zellen der Zellschichten, die auf der Membran vorhanden sind, gedehnt oder gestaucht werden. Dadurch ist es möglich, gezielt einen mechanischen Stress auf die Zellen einwirken zu lassen.

Die Aufgabe wird außerdem durch ein Halbzeug für eine Vorrichtung zur In-vitro-Herstellung und Kultivierung von Zellschichten gelöst. Diese Halbzeuge weisen zwei übereinander angeordnete und durch eine poröse Membran, mit zwei Seitenflächen, voneinander getrennte erste und zweite Kavitäten auf, wobei die erste Kavität in der Oberseite und die zweite Kavität in der Unterseite einer Grundplatte ausgebildet sind und durch eine der Seitenflächen der Membran eine zweite Wand als ein zweites Zellsubstrat der ersten Kavität und durch die andere der Seitenflächen eine zweite Wand als ein drittes Zellsubstrat der zweiten Kavität gebildet ist. Die erste Kavität ist zu ihren Enden hin spitz zulaufend und in ihrem mittleren Teil rechteckig ausgebildet und eines der spitz auslaufenden Enden mündet in eine erste Einlassöffnung zum Einlass eines ersten Fluidstroms eines ersten Fluids in die erste Kavität und das andere der spitz auslaufenden Enden mündet in eine erste Auslassöffnung zum Auslass des ersten Fluidstroms aus der ersten Kavität, wodurch eine laminare Strömung des ersten Fluidstromes bewirkt wird. Die zweite Kavität ist rechteckförmig geformt und ist mit einer zweiten Einlassöffnung zum Einlass eines zweiten Fluidstroms in die zweite Kavität und einer zweiten Auslassöffnung zum Auslass des zweiten Fluidstroms aus der zweiten Kavität durch kanalartige Verengungen verbunden.

Die Fluide können beispielsweise Zellsuspensionen und/oder Nährmedien sein. Ihre Eigenschaften, wie beispielsweise ihre chemische und/oder physikalische Zusammensetzung, ihre Temperatur, Viskosität, können zeitlich geregelt oder gesteuert verändert werden.

Halbzeuge sind gegenständliche Produkte, die erst nach einer weiteren Bearbeitung und / oder einer Montage mit anderen Halbzeugen oder Vorrichtungen eine selbständige technische Einheit darstellen.

Es ist möglich, die Kavitäten eines Halbzeugs mit einer flüssigkeitsdichten, vorzugsweise mit einer flüssigkeits- und gasdichten Abdichtung zu versehen, durch die die Kavitäten gegenüber der Umgebung abgedichtet sind. Durch die flüssigkeitsdichte Abdichtung mit einer Folie (Bonding) ist aus dem Halbzeug mindestens eine Kammer gebildet, die durch die Membran in wenigstens eine obere und eine untere Kammerhälfte, entsprechend der ersten und zweiten Kavität, getrennt ist.

Eine Vorrichtung zur In-vitro-Herstellung und Kultivierung von Zellschichten ist vorzugsweise dadurch gekennzeichnet, dass entweder zwei oder mindestens drei Halbzeuge übereinander montiert und entlang von aneinander anliegenden Rändern der ersten und zweiten Kavitäten flüssigkeitsdicht miteinander verbunden sind und jeweils die nach außen in eine Umgebung der Vorrichtung weisenden Kavitäten durch eine Wand überdeckt und gegenüber der Umgebung flüssigkeitsdicht abgedichtet sind.

In einer weiterführenden und vorteilhaften Ausgestaltung der Vorrichtung kann diese Messpunkte zur fluoreszenzbasierten und kontaktlosen Erfassung von Messwerten aufweisen. Mittels dieser Messpunkte sind beispielsweise die bereits weiter oben beschriebenen Verfahren ausführbar.

In einer sehr günstigen Weiterentwicklung der Vorrichtung ist jede der Einlassöffnungen mit einer Auslassöffnung einer anderen Kammer strömungstechnisch verbindbar, sodass erste und zweite Fluidströme durch mehrere erste und / oder zweite Kavitäten fließen. Durch eine solche Gestaltung sind Verschaltungen mehrerer Kavitäten durch einen Benutzer ermöglicht.

Die erfindungsgemäßen Halbzeuge können zu einer Vorrichtung zur In-vitro-Herstellung und Kultivierung von Zellschichten montiert werden, indem zwei der Halbzeuge übereinander montiert und entlang von aneinander anliegenden Rändern der ersten und zweiten Kavitäten flüssigkeitsdicht miteinander verbunden werden und jeweils die nach außen in eine Umgebung der Vorrichtung weisenden Kavitäten durch eine Wand überdeckt und gegenüber der Umgebung flüssigkeitsdicht abgedichtet werden.

Eine flüssigkeitsdichte Abdichtung gegenüber der Umgebung ist beispielsweise mittels einer Folie möglich, die über die entsprechende Kavität gebracht und mit Rändern der Kavität verbunden, beispielsweise geklebt oder gebondet wird.

Es ist auch möglich, dass mindestens drei der Halbzeuge übereinander montiert und entlang von aneinander anliegenden Rändern der ersten und zweiten Kavitäten flüssigkeitsdicht miteinander verbunden werden und jeweils die nach außen in eine Umgebung der Vorrichtung weisenden Kavitäten durch eine Wand überdeckt und gegenüber der Umgebung flüssigkeitsdicht abgedichtet werden.

Die erfindungsgemäßen Vorrichtungen können auch als ein Chip, auch Biochip genannt, ausgeführt sein. Dabei ist wenigstens eine Kavität auf einem Träger vorhanden, der beispielsweise die Abmessungen eines Objektträgers aufweist, wie er aus der Lichtmikroskopie bekannt ist.

Der Chip besteht aus mindestens einer Kavität, die durch ein Mikrokanalsystem mit anderen Kavitäten auf dem gleichen Chip oder auf weiteren Chips verbunden werden kann. In jeder Kavität kann ein Fluidstrom erzeugt werden, indem ein Fluid, wie beispielsweise eine Zellsuspension oder eine Lösung mit Nährstoffen, von der Einlassöffnung durch die Kavität zur Auslassöffnung strömt. Aufgrund der vorhandenen Membran können Zellschichten auf der Membran jeder Kavität zusätzlich durch ein unteres Mikrokanalsystem unabhängig von anderen Kavitäten auf dem Biochip mit Nährmedium versorgt werden. In jeder Kavität befindet sich eine Membran, die porös und freitragend ist und die in ihrer Materialbeschaffenheit und der verwendeten Porengröße und Porendichte so gewählt ist, dass eine vollständige Besiedelung der Membran mit Zellen möglich ist. Sehr vorteilhaft ist es, wenn die Membranbeschaffenheit zugleich eine lichtmikroskopische Beobachtung der Zellen ohne eine erhöhte Lichtabsorption oder Lichtstreuung ermöglicht. Dies ist beispielsweise dadurch gegeben, dass die Membran transparent ist, was beispielsweise aber nicht ausschließlich durch Materialien wie Polyester (PET) oder Polykarbonat (PC) oder Polydimethylsiloxan (PDMS) erreicht ist.

Der Chip besteht vorzugsweise aus biokompatiblem Material wie beispielsweise zyklischen Olefin-Polymeren (zum Beispiel ZEONOR® oder TOPAS®). Die eingesetzten Materialen erlauben eine fluoreszenzmikroskopische Analyse der im Chip befindlichen Zellen oder Gewebe ohne störende Eigenfluoreszenz.

Durch die erfindungsgemäßen Halbzeuge ist vorteilhaft die Möglichkeit geschaffen, in erfindungsgemäßen Vorrichtungen Zellschichten, die sich auf der Membran bzw. auf den Membranen befinden, durch die Membran mit Nährstoffen zu versorgen. Diese Versorgung, die auch als Basisperfusion oder basale Perfusion bezeichnet werden kann, erlaubt auch den Aufbau dicker und komplexer Anordnungen von Zellschichten bei gleichzeitiger Sicherstellung der Versorgung auch der untersten, membrannahen Zellschichten.

Es ist möglich, dass ein Medium von der Auslassöffnung einer Kavität ganz oder teilweise wieder zu der Einlassöffnung der Kavität geführt ist. Durch einen solchen strömungstechnischen Kurzschluss kann beispielsweise eine basale Perfusion mit einer begrenzten Menge an Medium erreicht werden.

Die vorliegende Erfindung beschreibt ein mikrofluidisches Chipsystem, das vorzugsweise im standardisierten Objektträger-Design ausgeführt ist. Hierdurch wird eine Nutzung mit im Labor verbreiteter Standard-Gerätetechnik für Liquid Handling und Analytik möglich. Das System vereinigt die jeweiligen Vorteile der bestehenden Systeme, wie des Zellkultur-Inserts (Transwell© System) und der Boyden-Kammer. Aufgrund der Ausführung in der Konstruktion sind zwei einzelne Chips miteinander zu einem Kammerkörper mit zwei Membranen kombinierbar. Das System erlaubt dabei die Kultivierung von bis zu vier voneinander getrennten Zell- oder Gewebeschichten und die Perfusion des Systems über bis zu drei unabhängige Kanalsysteme. Aufgrund der Ausführung der Vorrichtung werden nur geringe Volumina für Untersuchungen benötigt. Weiterhin sind berührungs- und zerstörungsfreie Untersuchungen der zellulären Prozesse innerhalb des Biochips, z. B. detaillierte mikroskopische und/oder spektroskopische Analysen, möglich. Der Chip dient als Basis für die Gestaltung von Nachbildungen des Aufbaus und der Funktion der Blutgefäße, der Leber, des Darms und eines Tumormodells. Diese konkreten Ausgestaltungsmerkmale und abstrahierten Modelle von Organstrukturen und deren Funktionsabbildung im mikrofluidischen Chip sind hier offenbart.

Mit erfindungsgemäßen Vorrichtungen sowie unter Anwendung des beschriebenen Verfahrens ist es beispielsweise möglich, verschiedene Gewebe oder gar Organoide dynamisch zu verbinden. Beispielsweise kann in einer Kavität bzw. in einer erfindungsgemäßen Vorrichtung mit zwei oder mehr Kavitäten ein Gewebe oder ein Organoid vorhanden sein. Diese Kavitäten sind mit weiteren Kavitäten verbunden, indem deren Auslassöffnungen durch Leitungen mit Einlassöffnungen anderer Kavitäten verbunden sind. In den anderen Kavitäten können gleiche oder andere Gewebe oder Organoide vorhanden sein. Ein Medium, das eine Kavität mit einem Gewebe oder Organoid durchströmt hat, gelangt anschließend in eine andere Kavität, in der ein gleiches oder ein anderes Gewebe oder Organoid vorhanden ist. Beispielsweise ist es möglich, dass eine Leber-Darm-Achse aufgebaut ist, indem in einer ersten erfindungsgemäßen Vorrichtung Zellschichten der Leber vorhanden sind und in einer zweiten erfindungsgemäßen Vorrichtung Zellschichten des Darms vorhanden sind. Ein Medium oder, je nach Ausführung und experimenteller Gestaltung der Leber-Darm-Achse, mehrere Medien gelangen nach dem Durchströmen der ersten Vorrichtung in die zweite Vorrichtung. Durch eine solche Anordnung können physiologische Abläufe künstlich und auf kleinem Raum nachempfunden werden.

Mittels der erfindungsgemäßen Vorrichtungen und des beschriebenen Verfahrens ist die Nachbildung komplexer organoider Strukturen, wie beispielsweise, aber nicht ausschließlich des Lebersinusoids, von Blutgefäßen, des Darms oder Strukturen von Tumoren und deren versorgenden Blutgefäßen möglich. Dabei können bestimmende Parameter der Zellkultur wie Nährmedium-Zusammensetzung, Fließgeschwindigkeit des perfundierten Mediums, Sauerstoff- und / oder Kohlendioxid-Sättigung, pH-Wert und Temperatur aufgrund der geringen Volumina leicht kontrollierbar sein. Da die die Zell- und Gewebeschichten versorgenden Fluidströme unabhängig voneinander steuerbar sind, können die vorgenannten Zellkulturparameter für jede Kavität einzeln eingestellt und verschiedene Gewebeschichten innerhalb einer Kavität unter verschiedenen Bedingungen perfundiert werden. Hierdurch wird eine optimale Anpassung an die einzelnen experimentellen Erfordernisse möglich. So können zum Beispiel Zell- oder Gewebeschichten mit unterschiedlichen Bedürfnissen an das Zellkulturmedium gleichzeitig in einer Kavität kultiviert und deren Interaktion untersucht werden.

Das System ist in einer vorteilhaften Ausführung mit einer Sensorik, beispielsweise für pH-Werte und partielle Gasdruckmessungen an dem Medium, ausrüstbar. Auf den Bondingfolien können weiterhin Elektroden vorhanden, beispielsweise aufgedampft sein, um über elektrische Widerstandsmessungen Rückschlüsse auf die Zellbesiedelungsdichte, Zellfunktion und / oder Zellvitalität auf den einzelnen Membranen zu erhalten. Die Chips können zudem modular über Schlauchsysteme miteinander seriell oder parallel verbunden werden, um so beispielweise die Interaktion verschiedener Organoide zu untersuchen.

Der Aufbau der Chips erlaubt die Konstruktion mehrschichtiger Zellanordnungen für die technische Abbildung einer Organstruktur und die Nachahmung dieser Organstruktur zum Zwecke der Diagnostik, Substanztestung oder Untersuchung molekularer Signalprozesse zwischen oder innerhalb von Zellen und Geweben. Innerhalb der Chips können Strukturen verschiedener Organe nachgestellt werden und diese über mikrofluidische Kreisläufe verbunden werden, so dass Kommunikationsprozesse des Körpers in der technischen Lösung des mikrofluidisch versorgten Chips nachempfunden werden können.

Ein Vorteil der Erfindung ist die Möglichkeit zur Abbildung von menschlichen Organen, wie beispielsweise, aber nicht ausschließlich der Leber, des Darms oder von Blutgefäßen in einem mikrofluidisch unterstützten Chip. Dieses Biochip-System, bestehend aus dem mikrofluidisch unterstützten Chip und einer Menge aus Zellen und / oder Geweben, die gemeinsam mindestens zwei und bevorzugt drei voneinander unabhängig und in einer gemeinsamen Kavität kultivierte Schichten bilden. Der Biochip ist für die biomedizinische Forschung zur Untersuchung zellulärer Aspekte von humanen Organoiden konzipiert. Anwendungsfelder umfassen beispielsweise, jedoch nicht ausschließlich, das Screening für pharmazeutische Wirkstoffe für Therapien, die Untersuchung von hepatischen Pathomechanismen vermittelt durch mikrobiologische Erreger oder Gefahrstoffe, die Testung von Nahrungsmittelzusätzen und die Entwicklung von Diagnostika. Das System ist für den Ersatz von Tierversuchen in der Human- und Veterinärmedizin konzipiert.

Die Erfindung wird nachfolgend anhand von Abbildungen und Ausführungsbeispielen näher erläutert. Dabei zeigen:
- Fig. 1: ein erstes Ausführungsbeispiel eines erfindungsgemäßen Halbzeugs in einer Draufsicht mit teilweise dargestellter Bondingfolie,
- Fig. 2: ein Halbzeug nach dem ersten Ausführungsbeispiel in einer Ansicht von unten mit Bondingfolie,
- Fig. 3: eine übersichtsweise Darstellung eines Lebersinusoids,
- Fig. 4: ein Beispiel hergestellter Zellschichten in einem Halbzeug mit Bondingfolie,
- Fig. 5: eine schematische Darstellung der hydraulischen Verknüpfung von vier Kavitäten,
- Fig. 6: ein erstes Ausführungsbeispiel einer erfindungsgemäßen Vorrichtung,
- Fig. 7: eine Schnittdarstellung des ersten Ausführungsbeispiels der erfindungsgemäßen Vorrichtung mit Zellschichten,
- Fig. 8: ein zweites Ausführungsbeispiel einer erfindungsgemäßen Vorrichtung mit Messpunkten,
- Fig. 9: eine Schnittdarstellung des zweiten Ausführungsbeispiels der erfindungsgemäßen Vorrichtung mit Zellschichten,
- Fig. 10: eine schematische Darstellung der Zellschichten in den Kavitäten des zweiten Ausführungsbeispiels der erfindungsgemäßen Vorrichtung und
- Fig. 11: eine schematisch Darstellung einer ersten Elektrodenanordnung.

In den folgenden Ausführungsbeispielen werden die Vorrichtungen und Elemente schematisch und vereinfachend gezeigt. Gleiche Bezugszeichen bezeichnen dabei gleiche technische Merkmale, soweit dies nicht anders beschrieben ist. Die Zuordnung von Zellschichten zu bestimmten Zellsubstraten erfolgt lediglich beispielhaft.

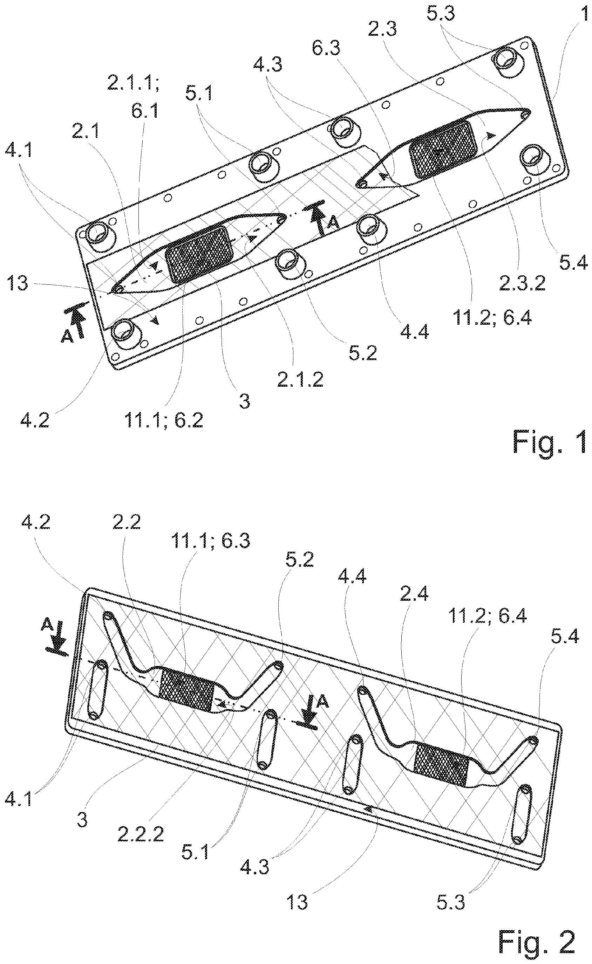

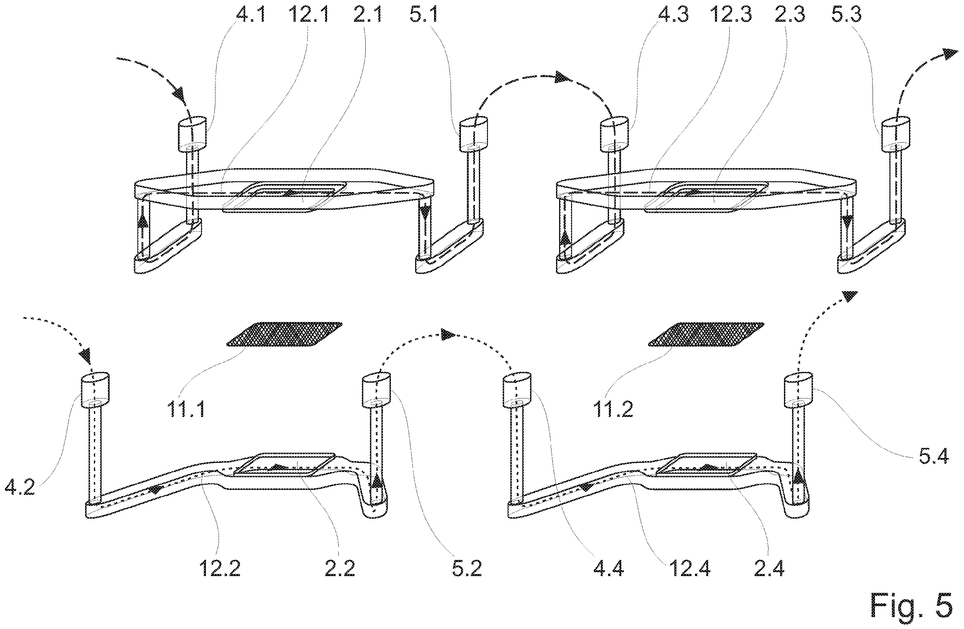

Als wesentliche Elemente eines ersten Ausführungsbeispiels eines erfindungsgemäßen Halbzeugs sind in Fig. 1 eine Grundplatte 1, eine erste Kavität 2.1 sowie eine erste Einlassöffnung 4.1 zum Einströmen eines ersten Fluidstroms 12.1 (siehe Fig. 5) in die erste Kavität 2.1 sowie eine erste Auslassöffnung 5.1 zum Ausströmen des ersten Fluidstroms 12.1 vorhanden. Zwischen der ersten Kavität 2.1 und einer zweiten Kavität 2.2 (siehe Fig. 2) ist eine erste Membran 11.1 vorhanden.

Die erste Kavität 2.1 ist zu ihren Enden hin spitz zulaufend und in ihrem mittleren Teil etwa rechteckig ausgebildet. In einem der spitz auslaufenden Enden mündet die erste Einlassöffnung 4.1, in dem anderen spitz auslaufenden Ende mündet die erste Auslassöffnung 5.1. Diese Gestaltung führt zu einer weitgehend laminaren Strömung des ersten Fluidstroms 12.1 durch die erste Kavität 2.1.

Auf der in Fig. 1 nicht sichtbaren Unterseite der Grundplatte 1 (siehe Fig. 2) ist die zweite Kavität 2.2 vorhanden. Eine zweite Einlassöffnung 4.2 zum Einströmen eines zweiten Fluidstroms 12.2 (siehe Fig. 5) in die zweite Kavität 2.2 sowie eine zweite Auslassöffnung 5.2 zum Ausströmen des zweiten Fluidstroms 12.2 sind auf der sichtbar dargestellten Oberseite vorhanden.

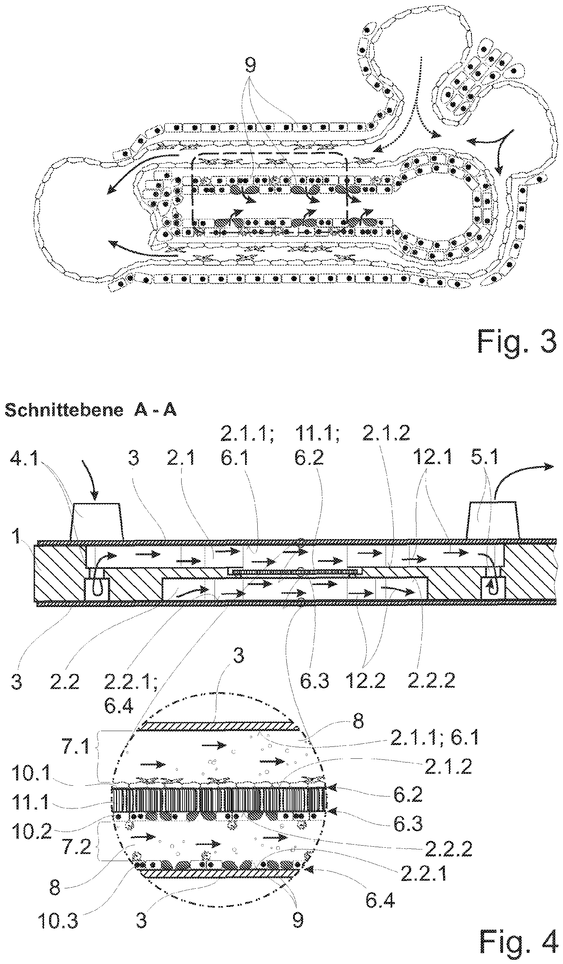

Eine Darstellung eines Schnittes entlang der Schnittebene A-A ist in Fig. 4 gezeigt und beschrieben.

In der Fig. 1 sind auf der Grundplatte 1 eine dritte Kavität 2.3 und eine vierte Kavität 2.4 (siehe Fig. 2) vorhanden. Ebenfalls sind eine dritte Einlassöffnung 4.3 zum Einströmen eines dritten Fluidstroms 12.3 (siehe Fig. 5) in die dritte Kavität 2.3 sowie eine dritte Auslassöffnung 5.3 zum Ausströmen des dritten Fluidstroms 12.3 und eine vierte Einlassöffnung 4.4 zum Einströmen eines vierten Fluidstroms 12.4 (siehe Fig. 5) in die vierte Kavität 2.4 sowie eine vierte Auslassöffnung 5.4 zum Ausströmen des vierten Fluidstroms 12.4 vorhanden. Alle Kavitäten 2.1 bis 2.4 sind von Rändern 13 umfangen, die durch die Grundplatte 1 gebildet sind.

Das Halbzeug ist in Fig. 1 in einer Ausgestaltung gezeigt, bei der die erste Kavität 2.1 und die dritte Kavität 2.3 durch eine Bondingfolie 3 (über der Kavität 2.3 nur teilweise dargestellt) gegenüber der Umgebung gas- und flüssigkeitsdicht abgedichtet ist.

Durch die Bondingfolie 3 ist in der ersten Kavität 2.1 eine erste Wand 2.1.1 gebildet, die ein erstes Zellsubstrat 6.1 ist. Die erste Membran 11.1 ist in einer zweiten Wand 2.1.2 gehalten. Die erste Membran 11.1 ist Teil der zweiten Wand 2.1.2 und stellt ein zweites Zellsubstrat 6.2 dar.

Zwischen der dritten und vierten Kavität 2.3, 2.4 ist eine zweite Membran 11.2 in der gleichen Weise wie soeben beschrieben angeordnet.

Nachfolgend wird vereinfachend auf die erste und zweite Kavität 2.1, 2.2 (Schnittebene A-A) und deren zugehörige Elemente Bezug genommen. Die technischen Elemente der dritten und vierten Kavitäten 2.3, 2.4 werden gegebenenfalls dargestellt und bezeichnet, jedoch nur ausdrücklich angesprochen, wenn dies für die Erläuterung erforderlich ist.

Die Unterseite der Grundplatte 1 ist in Fig. 2 gezeigt. Zu sehen ist die zweite Kavität 2.2 und die Unterseite der ersten Membran 11.1, die als ein drittes Zellsubstrat 6.3 dient. In dieser Perspektive ist eine zweite Wand 2.2.2 der zweiten Kavität 2.2 zu sehen, in der die erste Membran 11.1 gehalten ist und die durch die Rückseite der in Fig. 1 gezeigten zweiten Wand 2.1.2 der ersten Kavität 2.1 gebildet ist.

Die zweite Kavität 2.2 ist in ihrem mittleren Teil etwa rechteckig geformt, während sie mit der zweiten Einlassöffnung 4.2 und der zweiten Auslassöffnung 5.2 durch kanalartige Verengungen verbunden ist.

In weiteren Ausführungen des erfindungsgemäßen Halbzeugs können die Formen und Abmessungen der Kavitäten zueinander gleich sein.

Fig. 3 zeigt schematisch einen Lebersinusoid. Zu erkennen sind Zellen 9 unterschiedlicher Zelltypen und schichtweise Anordnungen der Zelltypen. Außerdem sind durch Pfeile Strömungsverhältnisse in dem Lebersinusoid gezeigt. Etwa in der Mitte ist ein gestrichelt umrahmter Bereich markiert, dessen Abfolge von Zellschichten mittels einer in Fig. 4 gezeigten Vorrichtung nachgebildet ist.

Die Vorrichtung gemäß Fig. 4 ist ein erfindungsgemäßes Halbzeug (siehe Fig. 1 und 2), das auf seiner Oberseite und seiner Unterseite je eine Bondingfolie 3 aufweist, durch die die erste und die zweite Kavität 2.1, 2.2 gegen die Umgebung abgedichtet sind. Durch die Bondingfolie 3 auf der Oberseite ist eine erste Wand 2.1.1 der ersten Kavität 2.1 gebildet, die als ein erstes Zellsubstrat 6.1 dient. Durch die Bondingfolie 3 auf der Unterseite ist eine erste Wand 2.2.1 der zweiten Kavität 2.2 gebildet, die als ein viertes Zellsubstrat 6.4 dient. Zwischen der ersten Wand 2.1.1 der ersten Kavität 2.1 und der zweiten Wand 2.1.2 der ersten Kavität 2.1 ist ein erster Spalt 7.1 vorhanden; zwischen der ersten Wand 2.2.1 der zweiten Kavität 2.2 und der zweiten Wand 2.2.2 der zweiten Kavität 2.2 ist ein zweiter Spalt 7.2 vorhanden.

In dem vergrößert gezeigten Ausschnitt sind eine erste bis dritte Zellschicht 10.1 bis 10.3 gezeigt, die auf dem zweiten, dritten und vierten Zellsubstrat 6.2; 6.3 und 6.4 vorhanden sind. Durch die erste bis dritte Zellschicht 10.1 bis 10.3, den ersten Spalt 7.1 und den zweiten Spalt 7.2 ist eine Anordnung von Zellschichten mit Zellen 9 nachgebildet, wie sie in Fig. 3 in dem umrahmten Bereich gezeigt ist.

In einer Möglichkeit zur Erzeugung der Anordnung von Zellschichten wird eine Zellsuspension 8 mit Zellen 9 des Zelltyps der ersten Zellschicht 10.1 in die erste Kavität 2.1 eingefüllt. Dabei ist die zu besiedelnde zweite Wand 2.1.2 der ersten Kavität 2.1 waagerecht ausgerichtet und befindet sich in einer unteren Lage, sodass durch Wirkung der Gravitation die Zellen 9 auf der zu besiedelnden zweiten Wand 2.1.2 der ersten Kavität 2.1 sedimentieren. Auf der als zweites Zellsubstrat 6.2 dienenden oberen Seite der ersten Membran 11.1 adhärieren die Zellen 9 und bilden die erste Zellschicht 10.1.

Danach wird eine Zellsuspension 8 mit Zellen 9 des Zelltyps der dritten Zellschicht 10.3 in die zweite Kavität 2.2 eingefüllt. Deren Lage bleibt unverändert. Dabei ist die nun zu besiedelnde erste Wand 2.2.1 der zweiten Kavität 2.2 waagerecht ausgerichtet und befindet sich in einer unteren Lage, sodass durch Wirkung der Gravitation die Zellen 9 auf der zu besiedelnden ersten Wand 2.2.1 der zweiten Kavität 2.2 sedimentieren. Auf der als viertes Zellsubstrat 6.4 dienenden ersten Wand 2.2.1 der zweiten Kavität 2.2 adhärieren die Zellen 9 und bilden die dritte Zellschicht 10.3.

Anschließend wird die Vorrichtung um 180 Grad gedreht, sodass nun die zweite Kavität 2.2 die obere Kavität ist. Die nun zu besiedelnde zweite Wand 2.2.2 der zweiten Kavität 2.2 ist waagerecht ausgerichtet und befindet sich nun in der unteren Lage, sodass durch Wirkung der Gravitation die Zellen 9 auf der zu besiedelnden zweiten Wand 2.2.2 der zweiten Kavität 2.2 sedimentieren. Auf der als drittes Zellsubstrat 6.3 dienenden Seite der ersten Membran 11.1 adhärieren die Zellen 9 und bilden die zweite Zellschicht 10.2.

Zur Erhaltung der bereits vorhandenen ersten Zellschicht 10.1 wird diese durch ein geeignetes Fluid, beispielsweise ein Nährmedium, in der ersten Kavität 2.1 versorgt.

Nach der Herstellung der Anordnung der drei Zellschichten 10.1 bis 10.3 ist die Anordnung der Zellschichten 10.1 bis 10.3 entsprechend dem markierten Bereich in Fig. 3 nachgebildet und ein Organoid geschaffen.

Die vier Kavitäten 2.1 bis 2.4 einer Grundplatte 1 können miteinander hydraulisch verknüpft sein, wie dies in Fig. 5 beispielhaft und schematisch gezeigt ist. Dabei ist die erste Auslassöffnung 5.1 mit der dritten Einlassöffnung 4.3 und die zweite Auslassöffnung 5.2 mit der vierten Einlassöffnung 4.4 verbunden, sodass ein erster Fluidstrom 12.1 aus der ersten Kavität 2.1 in die dritte Kavität 2.3 fließt und diese dort als dritter Fluidstrom 12.3 durchströmt. Ein zweiter Fluidstrom 12.2 durchströmt die zweite Kavität 2.2 und gelangt über die zweite Auslassöffnung 5.2 zur vierten Einlassöffnung 4.4 und von dort als vierter Fluidstrom 12.4 in die vierte Kavität 2.4.

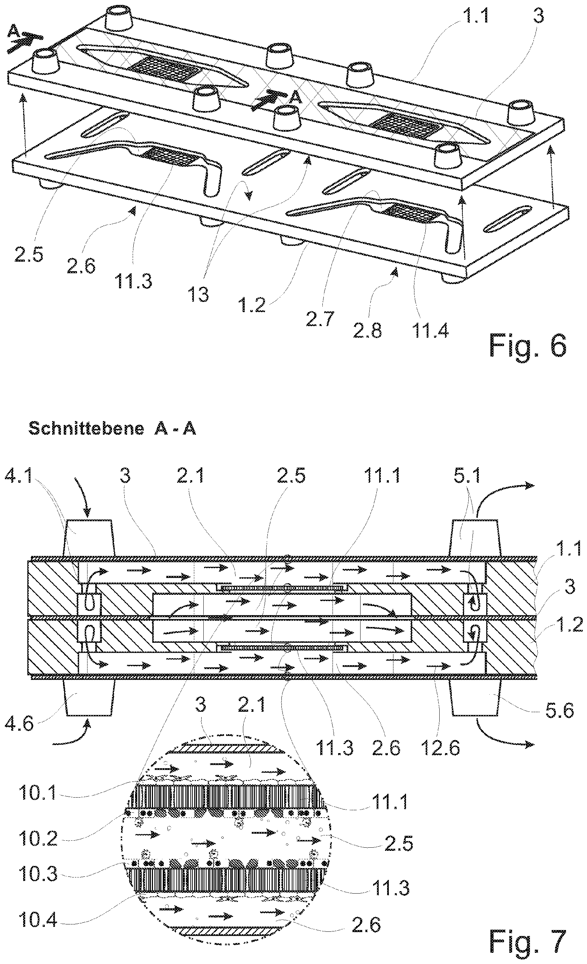

Das eben beschriebene Prinzip kann in weiteren Ausführungen der Erfindung auch zwischen Kavitäten verschiedener Grundplatten 1 oder Vorrichtungen (siehe z. B. Fig. 6 bis 9) verwendet sein. Außerdem können die Fluidströme 12.1 bis 12.4 anders geleitet sein. So kann in einer weiteren Ausgestaltung der Erfindung der erste Fluidstrom 12.1 in die vierte Kavität 2.4 und der zweite Fluidstrom 12.2 in die dritte Kavität 2.3 geleitet sein.

Es ist ferner möglich, dass die Fluidströme 12.1 bis 12.4 ineinander oder aufgeteilt und in unterschiedliche Kavitäten geführt sind. Auch ist es möglich, dass nur Anteile der Fluidströme 12.1 bis 12.4 in nachfolgende Kavitäten geleitet sind. So können Anteile der Fluidströme 12.1 bis 12.4 in die soeben durchströmten Kavitäten zurückgeleitet sein und diese erneut durchströmen.

Ein erstes Ausführungsbeispiel einer erfindungsgemäßen Vorrichtung ist in Fig. 6 dargestellt. Dabei sind eine erste Grundplatte 1.1 und eine zweite Grundplatte 1.2 mit ihren jeweiligen Unterseiten aneinander gesetzt, sodass sich die Ränder 13 der ersten Grundplatte 1.1 und zweiten Grundplatte 1.2 berühren (gezeigt ist ein Zustand während der Montage). Beide Grundplatten 1.1 und 1.2 sind an ihren jeweiligen Oberseiten durch Bondingfolien 3 abgedichtet, wie dies bereits vorstehend beschrieben ist. Die zweite Grundplatte 1.2 weist ebenfalls Kavitäten auf, die nach oben beschriebener Logik die fünfte bis achte Kavität 2.5 bis 2.8 sind (nur die fünfte Kavität 2.5 und die siebente Kavität 2.7 gezeigt; auf die sechste Kavität 2.6 und die achte Kavität 2.8 in Fig. 6 lediglich durch Pfeile hingewiesen). Die zweite Grundplatte 1.2 weist zudem eine dritte und eine vierte Membran 11.3 und 11.4 auf. Beide Grundplatten 1.1 und 1.2 sind flüssigkeitsdicht miteinander verbunden, beispielsweise geklebt. Die zweite Kavität 2.2 (siehe auch Fig. 1) und die vierte Kavität 2.4 (siehe auch Fig. 1) der Grundplatte 1.1 sind über die fünfte Kavität 2.5 bzw. über die siebente Kavität 2.7 der Grundplatte 1.2 gebracht und bilden jeweils eine gemeinsame Kavität, nämlich die fünfte Kavität 2.5 bzw. die siebente Kavität 2.7.

Der so geschaffene Aufbau der erfindungsgemäßen Vorrichtung ist in Fig. 7 als Schnittdarstellung entlang der Schnittebene A-A (Fig. 6) gezeigt. Durch die oben gezeigte erste Grundplatte 1.1 ist eine erste Kavität 2.1 gebildet. Diese ist durch die erste Membran 11.1 von der fünften Kavität 2.5 getrennt. Diese wiederum ist von der sechsten Kavität 2.6 durch die dritte Membran 11.3 getrennt. Die sechste Kavität 2.6 ist über die sechste Einlassöffnung 4.6 und die sechste Auslassöffnung 5.6 von einem sechsten Fluidstrom 12.6 durchströmt.

In der Ausschnittsvergrößerung sind die erste, die fünfte und die sechste Kavität 2.1, 2.5 und 2.6 sowie die erste Membran 11.1 und die dritte Membran 11.3 gezeigt. Auf den beiden Membranen 11.1 und 11.3 ist jeweils auf beiden Seiten eine Zellschicht 10.1 bis 10.4 vorhanden.

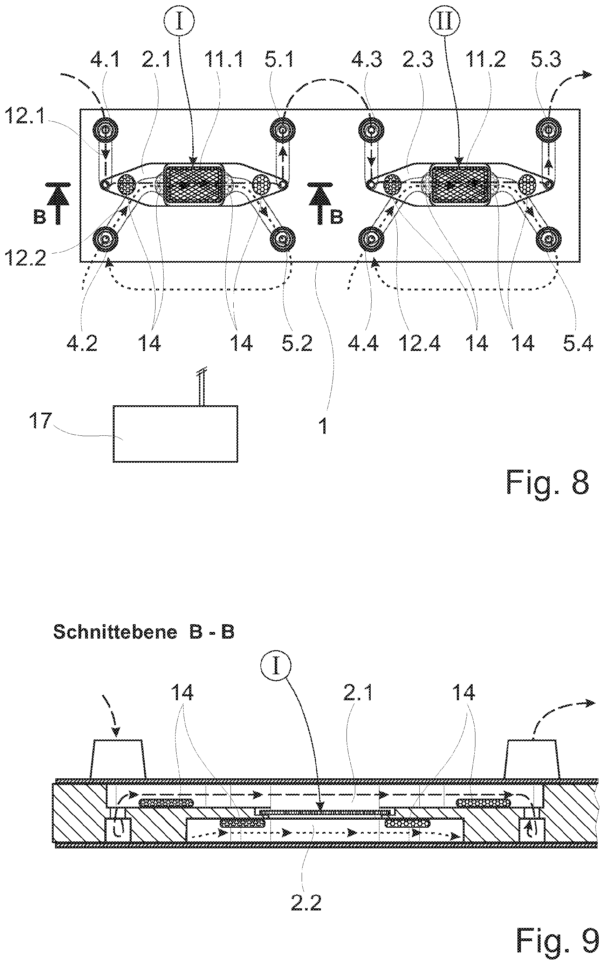

Ein zweites Ausführungsbeispiel einer erfindungsgemäßen Vorrichtung mit Messpunkten 14 ist in Fig. 8 gezeigt. Auf der gemäß Fig. 1 gestalteten Grundplatte 1 sind in jeder Kavität 2.1 bis 2.4 (nur die erste und die dritte Kavität 2.1, 2.3 gezeigt) Messpunkte 14 zur Erfassung von Messwerten aufgrund fluoreszierender Eigenschaften von Bestandteilen der Fluidströme 12.1 bis 12.4 (siehe z. B. Fig. 5) angeordnet. Diese sind datentechnisch mit einer Auswerte- und Steuereinheit 17 verbunden. Durch diese sind die Zusammensetzung, der Druck und die Temperatur der Fluidströme 12.1 bis 12.4 auf der Grundlage der erfassten Messwerte gesteuert einstellbar. Zudem werden die Messwerte für weitere Auswertungen wiederholt abrufbar gespeichert. Die Messpunkte 14 dienen der kontaktlosen Messung von Glukose und partiellem Sauerstoffverbrauch der Zellen 9 der Zellschichten.

In der ersten und zweiten Kavität 2.1 und 2.2 ist ein Organoid der Leber (Leberorganoid I) gemäß dem beschriebenen Verfahren geschaffen, während in den dritten und vierten Kavitäten 2.3 und 2.4 ein Organoid des Darms (Darmorganoid II) geschaffen ist. Die erste Auslassöffnung 5.1 ist hydraulisch mit der dritten Einlassöffnung 4.3 verbunden. Durch die zweite Einlassöffnung 4.2 wird der zweiten Kavität 2.2 ein zweiter Fluidstrom 12.2 zur Versorgung der Zellschichten in der zweiten Kavität 2.2 und der Zellschichten auf der ersten Membran 11.1 in der ersten Kavität 2.1 zugeführt. Der zweite Fluidstrom 12.2 ist über die zweite Auslassöffnung 5.2 abgeführt und wird in Anteilen mit frischem Fluid gemischt, bevor diese Mischung wieder über die zweite Einlassöffnung 4.2 in die zweite Kavität 2.2 gelangt. Entsprechendes gilt für die vierte Kavität 2.4. Durch die erste Einlassöffnung 4.1 wird ein erster Fluidstrom 12.1 durch die erste Kavität 2.1 hindurch zur ersten Auslassöffnung 5.1 geleitet. Von dort gelangt dieser über die dritte Einlassöffnung 4.3 in die dritte Kavität 2.3, durchströmt diese als dritter Fluidstrom 12.3 und wird über die dritte Auslassöffnung 5.3 abgeführt.

Durch diese Gestaltung sind Organoide und deren physiologische Wirkungen aufeinander in einem experimentellen Umfeld in vitro nachgebildet und können erfasst und bewertet werden.

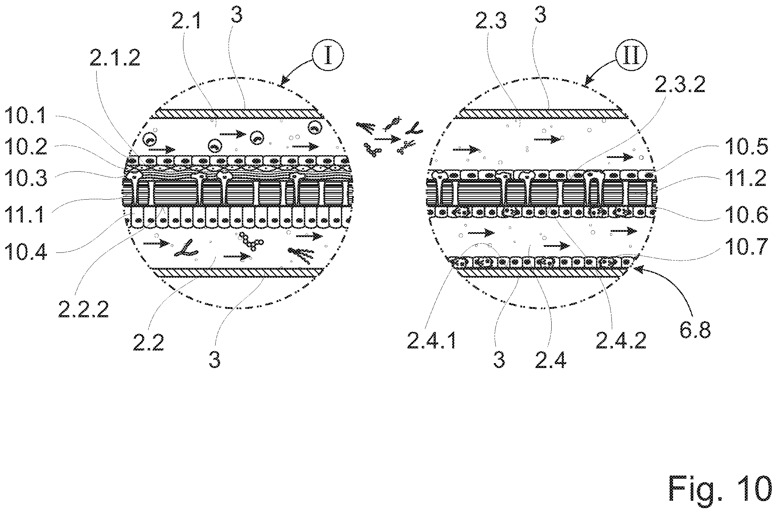

In Fig. 9 ist in einer Schnittdarstellung entlang der Schnittebene B - B (siehe Fig. 8) die Platzierung der Messpunkte 14 in der ersten Kavität 2.1 und in der zweiten Kavität 2.2 gezeigt. Durch die römischen Ziffern I und II sind Bereiche des Leberorganoids bzw. des Darmorganoids gekennzeichnet, die in Fig. 10 vergrößert dargestellt sind.

Das Leberorganoid I ist in der Fig. 10 in der linken Ausschnittsvergrößerung gezeigt. Auf der zweiten Wand 2.1.2 der ersten Kavität 2.1 auf der ersten Membran 11.1 ist eine erste Zellschicht 10.1 als eine Schicht HIMEC (Human Intestinal Microvascular Endothelial Cells) über einer zweiten Zellschicht 10.2 FB (Fibroblasten) und einer dritten Zellschicht 10.3 ECM (extrazelluläre Matrix) vorhanden. Auf der zweiten Wand 2.2.2 der zweiten Kavität 2.2 auf der ersten Membran 11.1 ist eine vierte Zellschicht 10.4 HCEC (Humane Colon Epithelial Cells, humane Darm-Epithelzellen) vorhanden.

Das Darmorganoid II ist in der Fig. 10 in der rechten Ausschnittsvergrößerung gezeigt. Auf der zweiten Wand 2.3.2 der dritten Kavität 2.3 auf der zweiten Membran 11.2 ist eine fünfte Zellschicht 10.5 als eine Schicht HSEC (human sinusoidal endothelial cell) vorhanden. Diese ist mit moMΦ Zellen (primary human monocyte derived macrophages) durchsetzt, die als Ko-Kultur angesiedelt wurden. Auf der zweiten Wand 2.4.2 der vierten Kavität 2.4 auf der zweiten Membran 11.2 ist eine sechste Zellschicht 10.6 HSC (human stellate cells) vorhanden. Auf der als achtes Zellsubstrat 6.8 dienenden ersten Wand 2.4.1 der vierten Kavität 2.4 ist als eine siebente Zellschicht 10.7 eine Schicht HHC (human differentiated hepatocytes) vorhanden, die von kokultivierten BC (bile canaliculi) durchzogen ist.

In weiteren Ausführungen können mehr Zellschichten 10.1, 10.2, ... vorhanden sein, die zudem Zellen 9 anderer Zelltypen, andere Abfolgen der Zellschichten und / oder andere Anordnungen der Zellschichten 10.1, 10.2, ... auf den verschiedenen Zellsubstraten 6.1, 6.2, ... aufweisen können.

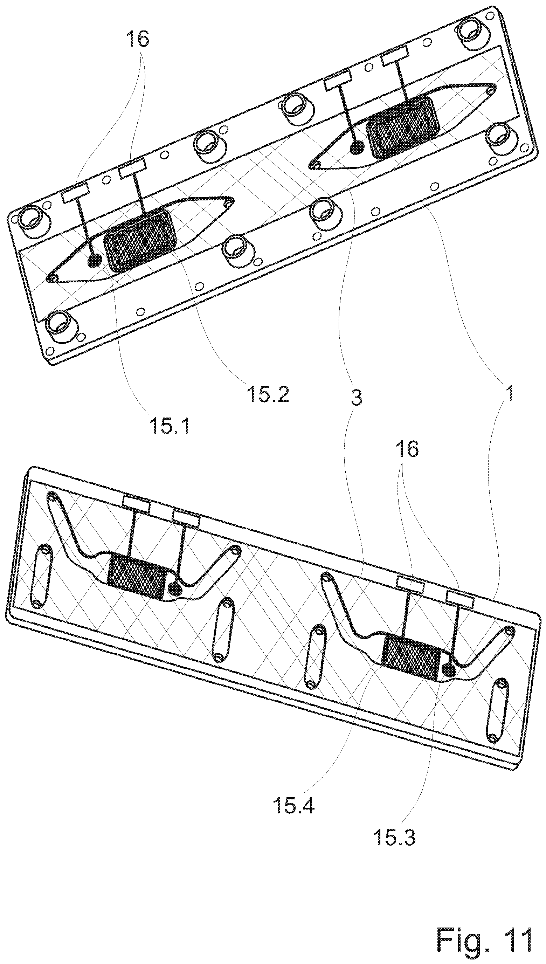

Eine Möglichkeit zur Erfassung von Messwerten an erfindungsgemäßen Halbzeugen und an erfindungsgemäßen Vorrichtungen ist in Fig. 11 vereinfacht gezeigt. Auf der Oberseite der Grundplatte 1 sind eine erste stromzuführende Elektrode 15.1 und eine erste Messelektrode 15.2 angeordnet. Auf der Unterseite der Grundplatte 1 sind eine zweite stromzuführende Elektrode 15.3 und eine zweite Messelektrode 15.4 angeordnet. Diese Elektroden 15.1 und 15.2 sowie 15.3 und 15.4 sind auf je eine Bondingfolie 3 aufgedruckt. In weiteren Ausführungen können diese auch in die Grundplatte 1 integriert oder auf diese aufgebracht, beispielsweise aufgedruckt sein. Jede Elektrode 15.1 bis 15.4 weist eine Kontaktierungsfläche 16 zur elektrischen und / oder messtechnischen Kontaktierung auf.

Durch Anlegen einer elektrischen Spannung an die Elektroden 15.1 bis 15.4 können Messwerte von erfassten elektrischen Spannungen, elektrischen Strömen, ohmschen Widerständen und / oder Impedanzen erhalten und ausgewertet werden.

Die Elektroden 15.1 bis 15.4 werden beispielsweise zur Impedanzmessung direkt oberhalb und unterhalb der ersten Membran 11.1 und / oder der zweiten Membran 11.2 auf den Bondingfolien 3 aufgebracht, beispielsweise gesintert oder gedruckt. Die Zuführung von Wechselstrom zum Aufbau eines elektrischen Feldes erfolgt auf die Kontaktierungsflächen 16. Dabei liegen sich die beiden stromzuführenden Elektroden 15.1 und 15.3 beispielsweise in der ersten Kavität 2.1 auf der flüssigkeitszuführenden Seite und in der zweiten Kavität 2.2 auf der flüssigkeitsabführenden Seite gegenüber.

### Bezugszeichenliste

- 1: Grundplatte
- 1.1: erste Grundplatte
- 1.2: zweite Grundplatte
- 2.1: erste Kavität
- 2.1.1: erste Wand (der ersten Kavität 2.1)
- 2.1.2: zweite Wand (der ersten Kavität 2.1)
- 2.2: zweite Kavität
- 2.2.1: erste Wand (der zweiten Kavität 2.2)
- 2.2.2: zweite Wand (der zweiten Kavität 2.2)
- 2.3: dritte Kavität
- 2.3.2: zweite Wand (der dritten Kavität 2.3)
- 2.4: vierte Kavität
- 2.4.1: erste Wand (der vierten Kavität 2.4)
- 2.4.2: zweite Wand (der vierten Kavität 2.4)
- 2.5: fünfte Kavität
- 2.6: sechste Kavität
- 2.7: siebente Kavität
- 2.8: achte Kavität
- 3: Bondingfolie
- 4.1: erste Einlassöffnung
- 4.2: zweite Einlassöffnung
- 4.3: dritte Einlassöffnung
- 4.4: vierte Einlassöffnung
- 4.6: sechste Einlassöffnung
- 5.1: erste Auslassöffnung
- 5.2: zweite Auslassöffnung
- 5.3: dritte Auslassöffnung
- 5.4: vierte Auslassöffnung
- 5.6: sechste Auslassöffnung
- 6.1: erstes Zellsubstrat
- 6.2: zweites Zellsubstrat
- 6.3: drittes Zellsubstrat
- 6.4: viertes Zellsubstrat
- 6.8: achtes Zellsubstrat
- 7.1: erster Spalt
- 7.2: zweiter Spalt
- 8: Zellsuspension
- 9: Zellen
- 10.1: erste Zellschicht
- 10.2: zweite Zellschicht
- 10.3: dritte Zellschicht
- 10.4: vierte Zellschicht
- 10.5: fünfte Zellschicht
- 10.6: sechste Zellschicht
- 10.7: siebente Zellschicht
- 11.1: erste Membran
- 11.2: zweite Membran
- 11.3: dritte Membran
- 11.4: vierte Membran
- 12.1: erster Fluidstrom
- 12.2.: zweiter Fluidstrom
- 12.3.: dritter Fluidstrom
- 12.4.: vierter Fluidstrom
- 12.6: sechster Fluidstrom
- 13: Ränder
- 14: Messpunkte
- 15.1: erste stromzuführende Elektrode
- 15.2: erste Messelektrode
- 15.3: zweite stromzuführende Elektrode
- 15.4: zweite Messelektrode
- 16: Kontaktierungsfläche
- 17: Auswerte- und Steuereinheit
- I: Leberorganoid
- II: Darmorganoid

## Patentansprüche

1. Halbzeug für eine Vorrichtung zur In-vitro-Herstellung und Kultivierung von Zellschichten (10.1, 10.2, ...) mit zwei übereinander angeordneten und durch eine poröse erste Membran (11.1), mit zwei Seitenflächen, voneinander getrennten ersten und zweiten Kavitäten (2.1, 2.2), wobei
- die erste Kavität (2.1) in der Oberseite und die zweite Kavität (2.2) in der Unterseite einer Grundplatte (1) ausgebildet sind,
- durch eine der Seitenflächen der ersten Membran (11.1) eine zweite Wand (2.1.2) als ein zweites Zellsubstrat (6.2) der ersten Kavität (2.1) und durch die andere der Seitenflächen eine zweite Wand (2.2.2) als ein drittes Zellsubstrat (6.3) der zweiten Kavität (2.2) gebildet ist,
- die erste Kavität (2.1) zu ihren Enden hin spitz zulaufend und in ihrem mittleren Teil rechteckig ausgebildet ist und eines der spitz auslaufenden Enden in eine erste Einlassöffnung (4.1) zum Einlass eines ersten Fluidstroms (12.1) in die erste Kavität (2.1) und das andere der spitz auslaufenden Enden in eine erste Auslassöffnung (5.1) zum Auslass des ersten Fluidstroms (12.1) aus der ersten Kavität (2.1) mündet, wodurch eine laminare Strömung des ersten Fluidstromes (12.1) bewirkt wird, und
- die zweite Kavität (2.2) rechteckförmig geformt und mit einer zweiten Einlassöffnung (4.2) zum Einlass eines zweiten Fluidstroms (12.2) in die zweite Kavität (2.2) und einer zweiten Auslassöffnung (5.2) zum Auslass des zweiten Fluidstroms (12.2) aus der zweiten Kavität (2.2) durch kanalartige Verengungen verbunden ist.

2. Halbzeug für eine Vorrichtung zur In-vitro-Herstellung und Kultivierung von Zellschichten (10.1, 10.2, ...) nach Anspruch 1, **dadurch gekennzeichnet,**
**dass** die Grundplatte (1) auf ihrer Oberseite und ihrer Unterseite je eine Bondingfolie (3) aufweist, durch die die erste und die zweite Kavität (2.1, 2.2) gegen die Umgebung abgedichtet sind.

3. Halbzeug für eine Vorrichtung zur In-vitro-Herstellung und Kultivierung von Zellschichten (10.1, 10.2, ...) nach Anspruch 1, **dadurch gekennzeichnet,**
**dass** die Grundplatte vier Kavitäten (2.1 - 2.4) aufweist, die hydraulisch miteinander verknüpft sind.

4. Vorrichtung zur In-vitro-Herstellung und Kultivierung von Zellschichten (10.1, 10.2, ...), bei der
- entweder zwei oder mindestens drei Halbzeuge nach Anspruch 1 übereinander montiert und entlang von aneinander anliegenden Rändern (13) der ersten und zweiten Kavitäten (2.1, 2.2) flüssigkeitsdicht miteinander verbunden sind und
- jeweils die nach außen in eine Umgebung der Vorrichtung weisenden Kavitäten (2.1, 2.2) durch eine erste Wand (2.1.1, 2.2.1) überdeckt und gegenüber der Umgebung flüssigkeitsdicht abgedichtet sind.

5. Vorrichtung zur In-vitro-Herstellung und Kultivierung von Zellschichten (10.1, 10.2, ...) nach Anspruch 4, **dadurch gekennzeichnet, dass** die Vorrichtung Messpunkte (14) zur fluoreszenzbasierten und kontaktlosen Erfassung von Messwerten aufweist.

6. Vorrichtung zur In-vitro-Herstellung und Kultivierung von Zellschichten (10.1, 10.2, ...) nach Anspruch 4 oder 5, **dadurch gekennzeichnet, dass** jede der Einlassöffnungen (4.1, 4.2) mit einer Auslassöffnung (5.1, 5.2) einer anderen Kavität strömungstechnisch verbindbar ist.

7. Vorrichtung zur In-vitro-Herstellung und Kultivierung von Zellschichten (10.1, 10.2, ...) nach Anspruch 4, **dadurch gekennzeichnet, dass** zwei Halbzeuge übereinander montiert werden, wobei die Grundplatten (1.1, 1.2) mit ihren jeweiligen Unterseiten aneinandergesetzt sind und die Oberseiten durch Bondingfolien (3) abgedichtet sind.

8. Vorrichtung zur In-vitro-Herstellung und Kultivierung von Zellschichten (10.1, 10.2, ...) nach Anspruch 4, **dadurch gekennzeichnet, dass** sie als mikrofluidisches Chipsystem in einem standardisierten Objektträgerdesign ausgeführt ist.

## Claims

1. A semi-finished product for a device for the in-vitro production and culturing of cell layers (10.1, 10.2, ...) with two first and second cavities (2.1, 2.2) arranged one above the other and separated from one another by a porous first membrane (11.1) with two lateral surfaces, wherein
- the first cavity (2.1) is formed in the upper surface and the second cavity (2.2) is formed in the lower surface of a base plate (1),
- a second wall (2.1.2) is formed by one of the lateral surfaces of the first membrane (11.1) as a second cell substrate (6.2) of the first cavity (2.1) and a second wall (2.2.2) is formed by the other of the lateral surfaces as a third cell substrate (6.3) of the second cavity (2.2),
- the first cavity (2.1) is tapered towards its ends and is rectangular in its central portion, and one of the tapered ends terminates in a first inlet opening (4.1) for the inlet of a first fluid flow (12.1) into the first cavity (2.1) and the other of the tapered ends terminates in a first outlet opening (5.1) for the outlet of the first fluid flow (12.1) from the first cavity (2.1), thereby causing a laminar flow of the first fluid flow (12.1), and
- the second cavity (2.2) is rectangularly shaped and connected, by channel-like constrictions, to a second inlet opening (4.2) for the inlet of a second fluid stream (12.2) into the second cavity (2.2) and to a second outlet opening (5.2) for the outlet of the second fluid stream (12.2) from the second cavity (2.2).

2. The semi-finished product for a device for the in-vitro production and culturing of cell layers (10.1, 10.2, ...) according to claim 1, **characterised in that** the base plate (1) has a bonding foil (3) on each of its upper and lower surfaces, by means of which bonding foil (3) the first and second cavities (2.1, 2.2) are sealed with respect to the environment.

3. The semi-finished product for a device for the in-vitro production and culturing of cell layers (10.1, 10.2, ...) according to claim 1, **characterised in that** the base plate has four cavities (2.1 - 2.4) which are hydraulically linked with each other.

4. A device for the in-vitro production and culturing of cell layers (10.1, 10.2, ...), wherein
- either two or at least three semi-finished products according to claim 1 are mounted one above the other and are connected to one another in a liquid-tight manner along adjacent edges (13) of the first and second cavities (2.1, 2.2) and
- in each case the cavities (2.1, 2.2) facing outwards into an environment of the device are covered by a first wall (2.1.1, 2.2.1) and are sealed in a liquid-tight manner with respect to the environment.

5. The device for the in-vitro production and culturing of cell layers (10.1, 10.2, ...) according to claim 4, **characterised in that** the device comprises measuring points (14) for the fluorescence-based and contactless acquisition of measured values.

6. The device for the in-vitro production and culturing of cell layers (10.1, 10.2, ...) according to claim 4 or 5, **characterised in that** each of the inlet openings (4.1, 4.2) can be fluidically connected to an outlet opening (5.1, 5.2) of another cavity.

7. The device for the in-vitro production and culturing of cell layers (10.1, 10.2, ...) according to claim 4, **characterised in that** two semi-finished products are mounted one above the other, the base plates (1.1, 1.2) being placed with their respective lower surfaces against one another and their upper surfaces being sealed by bonding foils (3).

8. The device for the in-vitro production and culturing of cell layers (10.1, 10.2, ...) according to claim 4, **characterised in that** it is designed as a microfluidic chip system in a standardised slide design.

## Revendications

1. Demi-produit pour un dispositif pour la production et la culture in vitro de couches cellulaires (10.1, 10.2, ...) avec deux premières et deuxièmes cavités (2.1, 2.2) disposées l'une au-dessus de l'autre et séparées l'une de l'autre par une première membrane poreuse (11.1) avec deux surfaces latérales, dans lequel
- la première cavité (2.1) est formée dans la face supérieure et la deuxième cavité (2.2) est formée dans la face inférieure d'une plaque de base (1),
- une deuxième paroi (2.1.2) est formée par l'une des faces latérales de la première membrane (11.1) comme deuxième substrat cellulaire (6.2) de la première cavité (2.1) et une deuxième paroi (2.2.2) est formée par l'autre des faces latérales comme troisième substrat cellulaire (6.3) de la deuxième cavité (2.2),
- la première cavité (2.1) est pointue vers ses extrémités et est rectangulaire dans sa partie centrale, et l'une des extrémités pointues débouche dans une première ouverture d'entrée (4.1) pour l'entrée d'un premier courant de fluide (12.1) dans la première cavité (2.1) et l'autre des extrémités pointues débouche dans une première ouverture de sortie (5.1) pour la sortie du premier courant de fluide (12.1) de la première cavité (2.1), ce qui provoque un écoulement laminaire du premier courant de fluide (12.1), et
- la deuxième cavité (2.2) est de forme rectangulaire et est reliée, par des constrictions en forme de canaux, à une deuxième ouverture d'entrée (4.2) pour l'entrée d'un deuxiième courant de fluide (12.2) dans la deuxième cavité (2.2) et à une deuxième ouverture de sortie (5.2) pour la sortie du deuxiième courant de fluide (12.2) de la deuxième cavité (2.2).

2. Demi-produit pour un dispositif pour la production et la culture in vitro de couches cellulaires (10.1, 10.2, ...) selon la revendication 1, **caractérisé en ce que** la plaque de base (1) comporte sur sa face supérieure et sur sa face inférieure respectivement un film de liaison (3), au moyen duquel la première et la deuxième cavité (2.1, 2.2) sont scellées par rapport à l'environnement.

3. Demi-produit pour un dispositif pour la production et la culture in vitro de couches cellulaires (10.1, 10.2, ...) selon la revendication 1, **caractérisé en ce que** la plaque de base comporte quatre cavités (2.1 - 2.4) qui sont reliées hydrauliquement entre elles.

4. Dispositif pour la production et la culture in vitro de couches cellulaires (10.1, 10.2, ...), dans lequel
- soit deux ou au moins trois demi-produits selon la revendication 1 sont montés l'un au-dessus de l'autre et sont reliés entre eux de manière étanche aux liquides le long des bords (13) des première et deuxième cavités (2.1, 2.2) qui sont en contact l'un avec l'autre, et
- dans chaque cas les cavités (2.1, 2.2) orientées vers l'extérieur dans un environnement du dispositif sont recouvertes par une première paroi (2.1.1, 2.2.1) et sont scellées de manière étanche aux liquides par rapport à l'environnement.

5. Dispositif pour la production et la culture in vitro de couches cellulaires (10.1, 10.2, ...) selon la revendication 4, **caractérisé en ce que** le dispositif comporte des points de mesure (14) pour l'acquisition de valeurs mesurées par fluorescence et sans contact.

6. Dispositif pour la production et la culture in vitro de couches cellulaires (10.1, 10.2, ...) selon la revendication 4 ou 5, **caractérisé en ce que** chacune des ouvertures d'entrée (4.1, 4.2) peut être reliée fluidiquement à une ouverture de sortie (5.1, 5.2) d'une autre cavité.

7. Dispositif pour la production et la culture in vitro de couches cellulaires (10.1, 10.2, ...) selon la revendication 4, **caractérisé en ce que** deux demi-produits sont montés l'un au-dessus de l'autre, les plaques de base (1.1, 1.2) étant placées avec leurs faces inférieures respectives l'une contre l'autre et les faces supérieures étant scellées par des films de liaison (3).

8. Dispositif pour la production et la culture in vitro de couches cellulaires (10.1, 10.2, ...) selon la revendication 4, **caractérisé en ce qu'**il est réalisé sous forme d'un système de puces microfluidiques dans une conception de portes-objet standardisée.
